# EUROPÄISCHE PATENTSCHRIFT

(11) **EP 4 355 279 B1**
(45) Veröffentlichungstag und Bekanntmachung des Hinweises auf die Patenterteilung: **16.04.2025**
(21) Anmeldenummer: 22733602.1
(22) Anmeldetag: 13.06.2022
(51) Int. Cl.: A61F 13/10, A61F 13/08

(54) **VERBAND ZUM UMSCHLIESSEN EINES BEIN- ODER ARMBEREICHES EINES LEBEWESENS**
BANDAGE FOR ENCLOSING A LEG REGION OR ARM REGION OF A LIVING BEING
BANDAGE DESTINÉ À ENFERMER UNE RÉGION DE JAMBE OU UNE RÉGION DE BRAS D'UN ÊTRE VIVANT

(30) Priorität: 14.06.2021 DE 102021115370
(43) Veröffentlichungstag der Anmeldung: 24.04.2024
(73) Patentinhaber: KOB GmbH, 67752 Wolfstein (DE)
(72) Erfinder: CAMERER, Sebastian, 39100 Bolzano (IT)
(74) Vertreter: Paul Hartmann AG Patents & Licensing
(86) Internationale Anmeldenummer: PCT/EP2022/065979
(87) Internationale Veröffentlichungsnummer: WO 2022/263352

(56) Entgegenhaltungen:
- EP-B1- 2 663 268
- EP-B1- 3 512 478
- WO-A1-2018/108798
- DE-A1- 102018 107 576
- DE-U1- 202012 102 576
- GB-A- 2 218 638
- US-A- 5 437 621
- US-A1- 2011 224 594
- US-A1- 2013 131 572
- US-A1- 2014 121 627
- US-A1- 2019 008 686
- US-B2- 8 221 340

## Beschreibung

Die Erfindung betrifft einen Verband, insbesondere einen gebrauchsfertigen Sekundärverband zum Umschließen eines Bein- oder Armbereiches eines Lebewesens.

Die Verwendung von Sekundärverbänden zur Fixierung und zum Schutz von primären Wundauflagen bei medizinischen Behandlungen wie beispielsweise Wundbehandlungen ist bekannt. Verbände dieser Art finden Gebrauch in der stationären Versorgung wie beispielsweise Krankenhäusern, Pflegeheimen oder sonstigen Einrichtungen, sowie in der ambulanten Versorgung, beispielsweise der häuslichen Altenpflege. Es kommen dabei häufig Produkte zum Einsatz, wie z.B. Wickelverbände, Schlauchverbände und Polsterverbände, die zum Teil mehrlagig übereinander kombiniert werden müssen, die eine geschulte, fachliche Expertise erfordern und einen Gebrauch durch Laien ausschließt. Beispielhaft für solch eine Versorgung ist die eines Wickelverbandes zur Wundversorgung für Körperteile wie Beine oder Arme. Beim Anlegen muss das Verrutschen der primären Wundauflage sowie eine Entstehung von Falten vermieden werden, um Druckstellen vorzubeugen. Ferner muss der Verband zugleich ausreichend eng sowie gleichmäßig am Körperteil angelegt werden, ohne dabei eine gefährliche Überkomprimierung auszuüben. Ein nachträgliches Nachjustieren ist dadurch erschwert, da es eine erneute Wicklung oder ein komplettes Aufschneiden des Verbands erfordert. Dabei kann die primäre Wundauflage verrutschen oder beschädigt werden, was in einem insgesamt höheren Zeit- und Kostenaufwand DE202012102576 offenbart eine Veterinärbandage die möglichst einfach and die Gliedmaße angelegt werden kann sowie einen dauerhaften Sitz und definierten Kompressionsdruck gewährleistet.

Aufgabe der vorliegenden Erfindung ist es folglich, einen gebrauchsfertigen Verband, insbesondere einen gebrauchsfertigen Sekundärverband bereitzustellen, der die Vorteile von Polsterverband, Schlauchverband und elastischer Binde in einem Produkt vereint, eine einfache Handhabbarkeit aufweist, ohne dass speziell geschulte Fachkenntnisse beim Anlegen des Verbandes erforderlich sind, und der zugleich eine verbesserte Passfähigkeit bereitstellt und Faltenbildung vermeidet.

Diese Aufgabe wird durch einen Verband gemäß Patentanspruch 1 gelöst. Vorteilhafte Ausgestaltungen und Weiterbildungen der Erfindung sind in den Unteransprüchen beschrieben.

Insbesondere wird diese Aufgabe gelöst durch einen Verband zum Umschließen eines Bein- oder Armbereiches eines Lebewesens, mit: einem länglichen, schlauchförmigen Verbandsteil, der zumindest teilweise in Längsrichtung einfach geschlitzt ist, und der dazu angepasst ist, in einem angelegten Zustand den Bein- oder Armbereich elastisch zu umschließen; und einem Verschlussteil mit Verschlusselementen, der mit einem Ende des geschlitzten Bereiches des Verbandsteils fest verbunden ist, um den geschlitzten Bereich durch überlappende Verbindung der benachbarten Enden des geschlitzten Bereiches mittels der Verschlusselemente lösbar zu verschließen. Dabei ist vorgesehen, dass der Verbandsteil in Längsrichtung unterschiedliche Kompressionszonen mit jeweils unterschiedlichen radialen Kompressionsstärken quer zur Längsrichtung oder jeweils unterschiedlichen Elastizitätsmodulen des Verbandmaterials aufweist. Der Verbandsteil ist aus zumindest einem Zuschnitt (Z) eines Stretch-Materials hergestellt. Das Stretch-Material umfasst entweder Elastomer-Fäden mit unterschiedlicher Dicke, die unterschiedliche Kompressionsstärken in den jeweils unterschiedlichen Kompressionszonen erzeugen, oder Elastomer-Fäden mit unterschiedlicher Vorspannung, die unterschiedliche Kompressionsstärken in den jeweils unterschiedlichen Kompressionszonen (K1, K2) erzeugen.

Es ist also ein anpassungsfähiger, gebrauchsfertiger Waden-, Unterschenkel- oder Unterarmverband vorgesehen, der den Wadenbereich oder den Unterschenkel vom Knöchel bis zum Knie, oder den Unterarm vom Ellenbogen bis zur Hand, bedeckt.

Hierbei wird mittels der unterschiedlichen Kompressionszonen eine gute Anpassung des Verbandes an einen Bein- oder Armbereich realisiert, ohne dass ein spezielles Fachwissen beim Anlegen des Verbandes nötig ist.

Die unterschiedlichen Kompressionszonen können dabei vorbestimmten Bereichen eines Schenkels, insbesondere eines Unterschenkels, oder eines Arms, insbesondere eines Unterarms, zugeordnet sein.

In diesem Zusammenhang kann eine erste Kompressionszone in dem Verbandsteil einem Bundbereich am Knie und/oder am Knöchel des Unterschenkels zugeordnet sein und eine zweite Kompressionszone in dem Verbandsteil kann einem Wadenbereich des Unterschenkels zugeordnet sein. Die erste Kompressionszone kann dabei eine höhere Kompressionsstärke aufweisen als die zweite Kompressionszone. Hierdurch wird ein mögliches Verrutschen des Verbandes weiter reduziert, da der Verband an dem Knie und/oder an dem Knöchel mit einer höheren Kompression anliegt. Zugleich kann durch die zweite Kompressionszone ein zu festes Anliegen bzw. ein zu hohe Druckausübung des Verbandes, zum Beispiel auf eine Wunde vermieden werden.

Der Verbandsteil kann aus zumindest einem Zuschnitt eines flachen Werkstoffs hergestellt sein, der einen Vlieswerkstoff umfasst.

Der Verbandsteil ist aus zumindest einem Zuschnitt eines Stretch-Materials hergestellt

Das Stretch-Material kann dabei eine Dehnbarkeit von mindestens 50% und ein Rückstellfähigkeit von 30% bis 100% aufweisen.

In einer Ausführungsform kann das Stretch-Material einen 2-Wege-Stretch-Stoff aufweisen, wobei sich eine Stretch-Richtung in Quer-Richtung des Verbandes erstreckt.

In einer weiteren Ausführungsform kann das Stretch-Material einen 4-Wege-Stretch-Stoff aufweisen, wobei sich eine Stretch-Richtung in Querrichtung und eine weitere Stretch-Richtung in Längsrichtung des Verbandes erstreckt. Dadurch wird zusätzliche Flexibilität bereitgestellt. Je nach Anforderung an den Verband kann entweder der 2-Wege-Stretch-Stoff oder der 4-Wege-Stretch-Stoff verwendet werden.

Das Stretch-Material umfasst entweder Elastomer-Fäden oder Elastan-Fasern mit unterschiedlicher Dicke die unterschiedliche Kompressionsstärken in den jeweils un-terschiedlichen Kompressionszonen erzeugen. Mittels der unterschiedlich dick ausgebildeten Elastomer-Fäden können Zonen gebildet werden, in welchen die dickeren Fäden-Durchmesser eine höhere Kompressionsstärke zur Folge haben, als Zonen mit dünneren Fäden-Durchmessern.

Zusätzlich oder alternativ umfasst das Stretch-Material Elastomer-Fäden oder Elastan-Fasern mit unterschiedlicher Vorspannung die unterschiedliche Kompressionsstärken in den jeweils unterschiedlichen Kompressionszonen erzeugen.

Der Verbandsteil kann neben dem geschlitzten Bereich einen schlauchartig geschlossenen, distalen Bereich umfassen, welcher bei einem Anlegen des Verbandes über einen Fuß oder eine Hand des Lebewesens gezogen wird und im angelegten Zustand des Verbandes zu dem Fuß oder der Hand benachbart ist. Dies kann die Handhabung des Verbands erleichtern. Beispielsweise verhindert der geschlossene, distale Bereich beim Anlegen des Verbandes ein Wegrutschen über ein Knöchel oder Handgelenk, so dass der Verband einfach und schnell geschlossen werden kann.

Der Verbandsteil kann hierbei in einem angelegten Zustand in seinem schlauchartig geschlossenen, distalen Bereich einen Knöchelbereich eines Fußes und in seinem geschlitzten Bereich einen Wadenbereich eines Unterschenkels komprimierend umschließen.

Der Verschlussteil kann als Verschlusselemente einzelne, aus einem Klettverschlussband ausgestanzte Klettverschlussteile umfassen, die einzeln mit dem Verbandsteil verbunden sind. Die Klettverschlussteile ermöglichen einen einfachen und sicheren Verschluss der Enden des Verbandsteils im geschlitzten Bereich.

In einer weiteren Ausführungsform kann der Verschlussteil auch als einstückiges Multi-Klettverschlussteil ausgebildet sein, das auf seiner einen Seite in seinem Nicht-Klettbereich mit dem Verbandsteil verbunden ist und auf seiner gegenüberliegenden Seite in seinem Klettbereich vorspringende Verschlusselemente aufweist, die durch Stanzen des Klettverschlussbandes ausgebildet sind.

Ferner kann der Verbandsteil aus zwei gleichförmigen Zuschnitten hergestellt sein, die mittels einer in Nahtrichtung elastischen Naht miteinander verbunden sind.

Der Verbandsteil kann eine einem Wadenbereich eines Unterschenkels angepasste Form aufweisen, wodurch er noch passgenauer ausgebildet ist.

Weiterhin kann der Verband als hygienisches Einweg-Produkt ausgebildet sein.

Die Erfindung wird im Folgenden beispielsweise anhand der Zeichnungen näher erläutert. Es zeigen:
Fig. 1A eine schematische perspektivische Ansicht eines Verbandes gemäß einem Ausführungsbeispiel der Erfindung;
Fig. 1B eine schematische perspektivische Ansicht des Verbandes gemäß einem Ausführungsbeispiel der Erfindung im geschlossenen Zustand;
Fig. 1C eine schematische Seitenansicht eines Verbandsteils des Verbandes gemäß einem Ausführungsbeispiel der Erfindung;
Fig. 1D eine schematische Seitenansicht des Verbandsteils des Verbandes gemäß einem Ausführungsbeispiel der Erfindung;
Fig. 1E eine schematische Explosionszeichnung des Verbandes gemäß einem Ausführungsbeispiel der Erfindung;
Fig. 1F eine schematische perspektivische Ansicht des Verbandes gemäß einem weiteren Ausführungsbeispiel der Erfindung im geschlossenen Zustand;
Fig. 2 eine schematische Seitenansicht des Verbandes gemäß mehreren Ausführungsbeispielen der Erfindung;
Fig. 3 schematische Schnittmuster zur Herstellung des Verbandes gemäß mehreren Ausführungsbeispielen der Erfindung; und
Fig. 4 eine schematische Darstellung einer Herstellung des Verbandes gemäß einem Ausführungsbeispiel der Erfindung.

Im Folgenden werden für gleiche und gleichwirkende Bauteile dieselben Bezugszeichen verwendet.

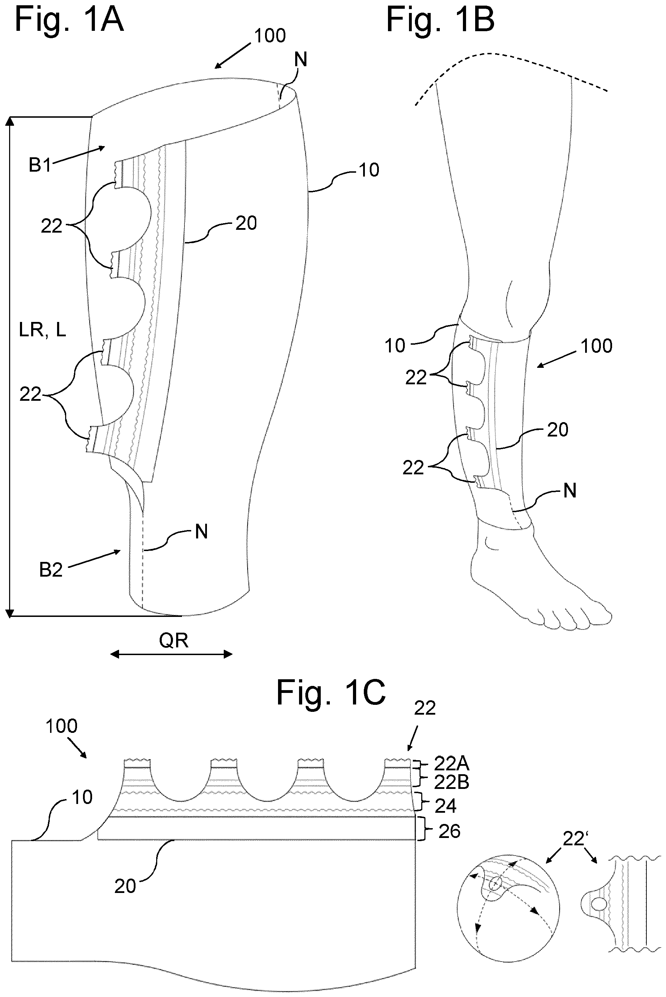

Fig. 1A zeigt eine schematische perspektivische Ansicht eines Verbandes 100 zum Umschließen eines Bein- oder Armbereiches eines Lebewesens. Bei dem Lebewesen kann es sich um einen Menschen, jedoch auch um ein Tier handeln. Der Verband 100 umfasst einen länglichen, schlauchförmigen Verbandsteil 10, der zumindest teilweise in Längsrichtung LR einfach geschlitzt ist. Das ist in Fig. 1A vereinfacht durch einen Pfeil auf den geschlitzten Bereich B1 angedeutet. Der Verbandsteil 10 umschließt in einem angelegten Zustand den Bein- oder Armbereich elastisch. In diesem Zusammenhang zeigt Fig. 1B den Verband 100 im geschlossenen Zustand, wobei beispielhalft ein Beinbereich illustriert ist. Ebenso möglich ist aber auch ein Armbereich.

Der Verband 100 umfasst weiterhin ein Verschlussteil 20 mit Verschlusselementen 22, 22'. Der Verschlussteil 20 ist mit einem Ende des geschlitzten Bereichs B1 des Verbandteils 10 fest verbunden, um den geschlitzten Bereich B1 durch überlappende Verbindung der benachbarten Enden des geschlitzten Bereiches B1 mittels der Verschlusselemente 22, 22' lösbar zu verschließen. Dies ist in Fig. 1A und Fig. 1B vereinfacht illustriert.

Die Länge des Schlitzes kann dabei je nach Anforderung an den Verband 100 variieren. Beispielsweise kann sie größer als 10%, oder größer als 20%, oder größer als 30%, oder größer als 40%, oder größer als 50%, oder größer als 60%, oder größer als 70%, oder größer als 80%, oder größer als 90%, oder größer als bis 100% der Gesamtlänge L des Verbandsteils 10 sein. Es ist weiter auch möglich, dass die Länge des Schlitzes bei 100% der Gesamtlänge L liegt, die Länge des geschlitzten Bereichs B1 also gleich der Länge LR entspricht, der schlauchförmige Verbandsteil 10 als vollständig in Längsrichtung L geschlitzt oder aufgeschnitten ist.

Der Verschlussteil 20 kann aus mehreren Elementen 22A, 22B, 24, 26 gebildet werden, was in Fig. 1C dargestellt ist. Fig. 1C zeigt eine schematische Seitenansicht des Verbandsteils 10, wobei ein Verschlusselement 22 aus einem Griffteil 22A und einem Hakenteil 22B, wie beispielsweise einem Klettverschlussteil hergestellt sein kann. Das Griffteil 22A erlaubt es einem Nutzer das Verschlusselement 22 zu greifen. In diesem Zusammenhang ist eine weitere Ausführungsform eines Verschlusselementes 22' auf der rechten Seite in der Fig. 1C gezeigt, das als Verschlusslasche mit einem Loch oder einer Ausnehmung in dem Hakenteil 22B ausgebildet ist. Die Ausnehmung kann hierbei kreisförmig oder oval oder elliptisch geformt sein. Hierdurch kann die Griffigkeit unterstützt und eine bessere Kontrolle für einen Finger des Nutzers bereitgestellt werden. Das Loch fungiert dabei als Grifföse für die Fingerkuppe. Diese Ausgestaltung ermöglicht ein flächiges, faltenfreies Anliegen auf gewölbten Oberflächen, was wiederrum in einer verbesserten Haftung und reduzierten Faltenbildung resultieren kann. Neben der in Fig. 1C dargestellten Verschlusslasche mit Loch oder ovaler Ausnehmung sind weitere Ausgestaltungen möglich. Der Verschlussteil 20 kann ferner einen elastischen Teil 24 und einen Verbindungsteil 26 aufweisen. Der Verbindungsteil 26 kann zum Beispiel den Verschlussteil 20 mittels Verkleben oder Verschweißen mit dem Verbandsteil 10 verbinden.

Ferner weist das Verbandsteil 10 in Längsrichtung LR unterschiedliche Kompressionszonen K1, K2 mit jeweils unterschiedlichen radialen Kompressionsstärken quer zur Längsrichtung LR (d.h. in Querrichtung QR) auf.

Hierdurch wird an entsprechenden Stellen des Bein- oder Armbereiches ein optimal angepasstes Anlegen des Verbandes 100 realisiert. Die Kompressionszonen K1, K2 ermöglichen eine vereinfachte Handhabung, da bei Anlegen keine speziellen Vorkenntnisse vonnöten sind und eine entsprechende Kompression an den jeweiligen Stellen vorgegeben ist. Ferner wird hierdurch eine Faltenbildung weiter verringert.

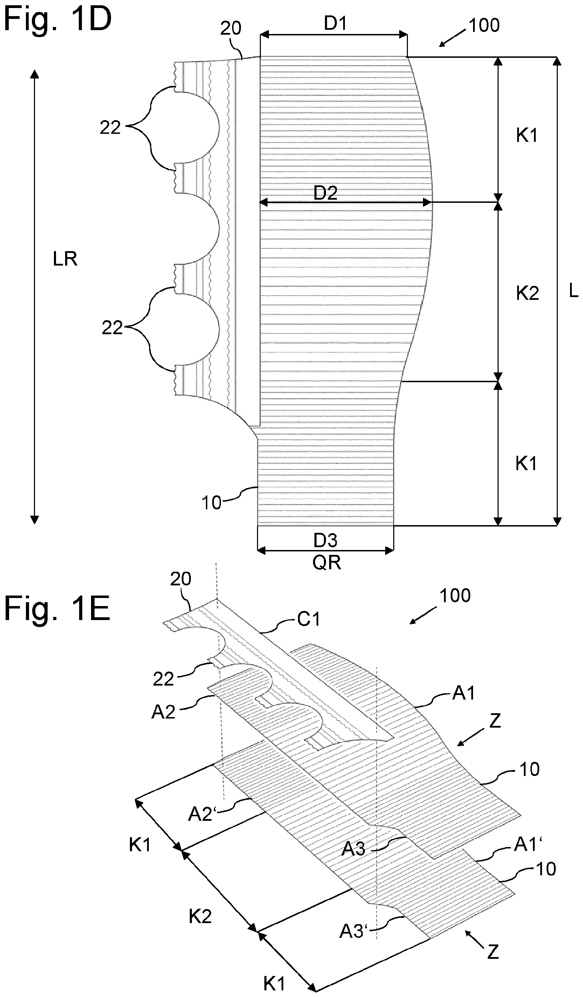

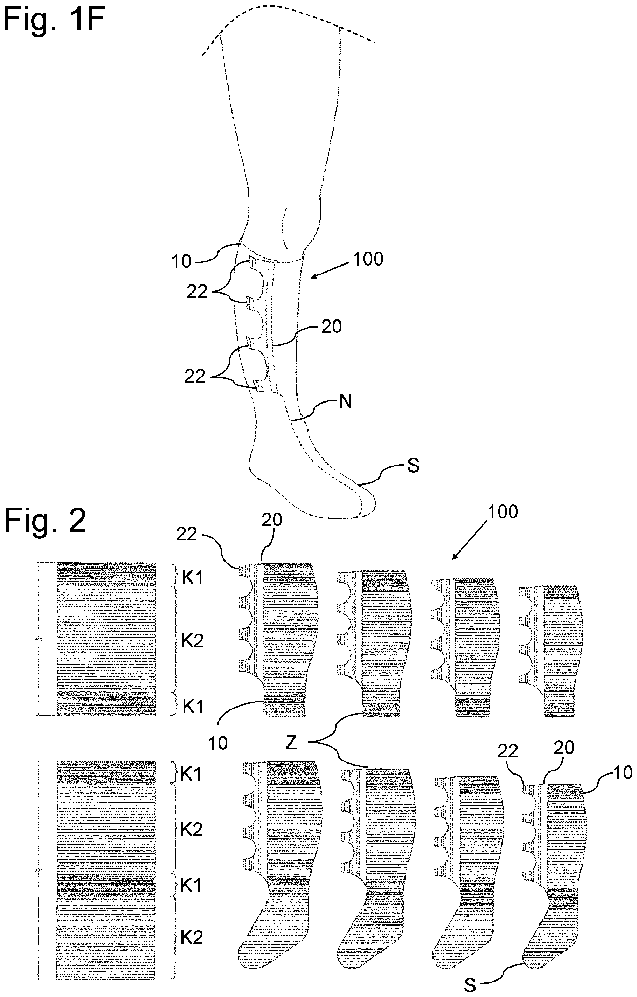

Die unterschiedlichen Kompressionszonen K1, K2 können beispielsweise vorbestimmten Bereichen eines Schenkels, insbesondere eines Unterschenkels, oder eines Arms, insbesondere eines Unterarms, zugeordnet sein. Fig. 1D zeigt eine schematische Seitenansicht des Verbandsteils 10, in welcher die Kompressionszonen K1, K2 illustriert sind. Wie aus Fig. 1D in Verbindung mit den Figuren 1A und 1B ersichtlich, kann eine erste Kompressionszone K1 in dem Verbandsteil 10 einem Bundbereich am Knie und/oder am Knöchel des Unterschenkels zugeordnet sein. Des Weiteren kann eine zweite Kompressionszone K2 in dem Verbandsteil 10 einem Wadenbereich des Unterschenkels zugeordnet sein. Hierbei kann die erste Kompressionszone K1 eine höhere Kompressionsstärke aufweisen als die zweite Kompressionszone K2, was vereinfacht durch die waagrechten Linien in Fig. 1D, die repräsentativ für entsprechende Materialfäden und deren Abstände zueinander stehen, gezeigt ist. Die elastischen Fäden, Elastan-Fäden, Elastan-Fasern oder elastomere Fäden werden später noch im Detail mit Bezugnahme auf Fig. 2 beschrieben.

Die Bemaßung des in Fig. 1D gezeigten Verbandteils 10 kann dabei unterschiedlich ausfallen. Ein oberer Durchmesser D1 kann zum Beispiel in einem Bereich von 50 mm bis 150 mm liegen. Ein mittlerer Durchmesser D2 kann in einem Bereich von 100 mm bis 200 mm liegen. Und ein unterer Durchmesser D3 kann in einem Bereich von 50 mm bis 150 mm liegen. Die Länge L des Verbandsteils 10 kann dabei im Bereich von 100 mm bis 400 mm liegen. Ein Längenverhältnis einer Kompressionszone K1, K2 zur Gesamtlänge L kann in einem Bereich zwischen 10% bis 70%, oder zwischen 10% bis 50%, oder zwischen 20% bis 40%, oder zwischen 25% und 35% liegen.

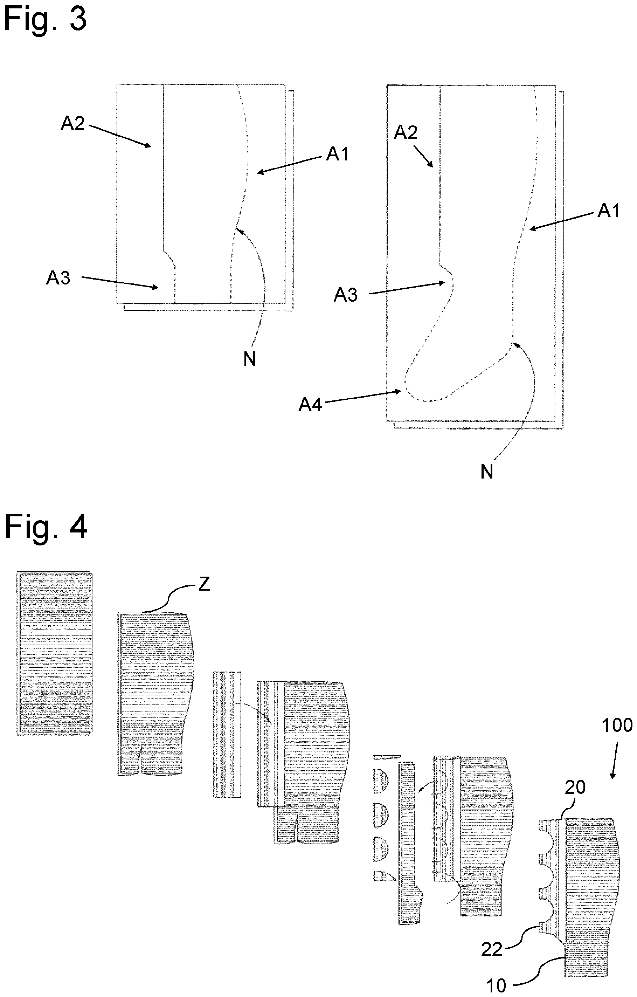

Fig. 1E zeigt eine schematische Explosionszeichnung des Verbandes 100, wobei ersichtlich ist, dass der Verbandsteil 10 aus zwei miteinander zu verbindenden gleichförmigen Zuschnitten Z gebildet werden kann, sowie dem oben beschriebenen Verschlussteil 20. Beide Zuschnitte Z umfassen die unterschiedlichen Kompressionszonen K1, K2 was vereinfacht in Fig. 1E durch Pfeile angedeutet ist. Die Zuschnitte Z können beispielsweise durch Ultraschallschweißen an entsprechenden Schnittkantenabschnitten A1, A1' miteinander verbunden werden. Die Schnittkantenabschnitte A1, A1' der jeweiligen Zuschnitte Z weisen eine gebogene oder gekrümmte Form auf, die in dem in Fig. 1E gezeigten Beispiel einem Wadenbereich oder Unterschenkel entsprechen und aus einem vorgegebenen Schnittmuster resultieren. Hierbei sind die Schnittkantenabschnitte S-förmig gekrümmt mit einem oberen konvexen Krümmungsbereich mit einem Krümmungsradius zwischen 300 mm und 450 mm im Wadenbereich und einem unteren konkaven Krümmungsbereich mit einem Krümmungsradius zwischen 250 mm und 350 mm , wobei der untere Krümmungsbereich dann in einen geraden Bündchenabschnitt im Knöchelbereich mündet. Die Schnittkantenabschnitte A2, A2' sind geradlinig und werden nicht miteinander verbunden. Sie bilden den geschlitzten Bereich B1 bzw. eine Öffnung. In dem oberen Zuschnitt Z wird der Schnittkantenabschnitt A1 jedoch mit einem ebenfalls geradlinig verlaufenden Schnittkantenabschnitt C1 des Verschlussteils 20 verbunden. Dies kann beispielsweise mittels des oben beschriebenen Verbindungsteils 26 erfolgen. Die Schnittkantenabschnitte A3, A3' werden ebenfalls miteinander verbunden und bilden einen geschlossenen, distalen Bereich B2, der später noch im Detail beschrieben wird. Wie aus Fig. 1E ersichtlich, weisen die Schnittkantenabschnitte A3, A3' ebenfalls eine aus dem Schnittmuster folgende gebogene oder gekrümmte Form auf, die im verbundenem Zustand einen Knöchel umschließen. Ebenso möglich ist es, den Verbandsteil 10 einstückig auszubilden. Die Herstellung des Verbandes 100 wird unter Bezugnahme auf die Figuren 3 und 4 später noch im Detail erläutert. Das Verhältnis der Längen des geschlitzten Bereichs B1 zu dem geschlossenen Bereich B2 kann hierbei in einem Bereich zwischen 5 % und 40 %, oder zwischen 10 % und 30 % oder zwischen 15 % und 25 % liegen. In diesen Fällen lässt sich der geschlossene Bereich B2 leicht über den Fuß oder die Hand ziehen, während noch mindestens mehr als die Hälfte des Verbandes 100 mittels des Verschlussteils 20 mit variabler Kompression verschließbar ist. Rein theoretisch kann auch der Verbandsteil 10 in Längsrichtung L an seinen beiden Enden geschlossene Bereiche aufweisen, wobei der geschlitzte Bereich B1 dann zwischen diesen Bereichen verläuft. Da ein geschlossener Bereich im Bundbereich des Knies ein Anlegen des Verbandes eher erschwert (und über den Wundbereich gezogen werden muss), soll diese Ausführungsform zwar von der Erfindung mitumfasst sein, jedoch nicht als vorzugsmäßige Ausführungsform.

Ein Verhältnis der ersten Kompressionsstärke der ersten Kompressionszone K1 zur zweiten Kompressionsstärke der zweiten Kompressionszone K2 kann dabei größer 1,5, oder größer 2, oder größer 3, oder größer 3,5, oder größer 4, oder größer 4,5 sein. Die Kompressionsstärke der ersten Kompressionszone K1 an einem Bundbereich, wie dem in Fig. 1A gezeigten Kniebereich und Knöchelbereich gleich sein. Sie kann aber auch unterschiedlich sein. Die Anzahl der Kompressionszonen in den in den Figuren 1D, 1E, 2, und 4 gezeigten Beispielen ist beispielhaft und weitere Kompressionszonen mit unterschiedlichen Kompressionsstärken sind möglich. Obwohl es also bevorzugt ist, dass die Kompressionsstärke der ersten Kompressionszone K1 sowohl im Bundbereich am Knie als auch am Knöchel gleich ist, kann statt der ersten Kompressionszone K1 eine dritte Kompressionszone im Bundbereich am Knöchel vorgesehen werden, dessen Kompressionsstärke von der Kompressionsstärke im Bundbereich am Knie unterschiedlich ist oder von ihr abweicht. Jedoch ist im diesem Fall die Kompressionsstärke dieser dritten Kompressionszone ebenfalls größer als die Kompressionsstärke der zweiten Kompressionszone K2. Unter dem Begriff Kompressionsstärke soll eine Kraft oder ein Druck verstanden werden, die oder der bei einem Anlegen des Verbandes aufgrund einer umfangsmäßigen Elastizität oder Rückstellkraft innerhalb des Verbandes (beispielsweise durch eingebrachte Elastanfasern oder elastomere Fäden in Umfangsrichtung quer zur Längsrichtung L) radial nach innen auf das vom Verband umschlossene Körperteil wirkt. Im Bereich der Kompressionsstrümpfe werden für die Kompressionsstärke im Sinne der Erfindung Kompressionsklassen CCL1 (mäßige Kompression mit einem Kompressionsdruck von 2,4 kPA bis 2,8 kPA), CCL2 (mittelkräftige Kompression mit einem Kompressionsdruck von 3,1 kPA bis 4,3 kPA), CCL3 (kräftige Kompression mit einem Kompressionsdruck von 4,5 kPA bis 6,1 kPA) und CCL4 (extrakräftige Kompression mit einem Kompressionsdruck von größer 6,5 kPA) verwendet. Im Bereich der Kompressionsstrümpfe werden für die Kompressionsstärke im Sinne der Erfindung Kompressionsklassen CCL1 (mäßige Kompression mit einem Kompressionsdruck von 2,4 kPa bis 2,8 kPa), CCL2 (mittelkräftige Kompression mit einem Kompressionsdruck von 3,1 kPa bis 4,3 kPa), CCL3 (kräftige Kompression mit einem Kompressionsdruck von 4,5 kPa bis 6,1 kPa) und CCL4 (extrakräftige Kompression mit einem Kompressionsdruck von größer 6,5 kPa) verwendet. Hierbei liegt die Kompressionsstärke der Kompressionszonen K1, K2 des erfindungsgemäßen Verbandes 100 vorzugsweise im Bereich der Kompressionsklasse CCL1, oder im Bereich der Kompressionsklasse CCL2, oder im Bereich der Kompressionsklasse CCL3, oder im Bereich der Kompressionsklasse CCL4, je nach Anwendung und Einsatzzweck des Verbandes 100. Der Begriff der Kompressionsstärke bezieht sich dabei stets auf eine Kompressionsstärke in einem angelegten Zustand des Verbandes an einem normal geformten Unterschenkel oder Unterarm, die aus einer der Kompressionsstärke entsprechenden Elastizität oder Dehnsteifigkeit in Umfangsrichtung des verschlossenen Verbandes 100 aufgrund einer Auslenk-Dehnung des Verbandmaterials resultiert. So ist also der Begriff der Kompressionsstärke als äquivalent anzusehen zu einem Elastizitätsmodul des Verbandmaterials (oder der elastischen Stützstruktur aus elastomeren Fäden) einer Kompressionszone K1, K2 in Quer-Richtung QR oder Umfangsrichtung des Verbandes 100.

In einer weiteren Ausführungsform kann der Verband 100 auch mit zusätzlichen Produkten zur medizinischen Behandlung, wie beispielsweise einem Fußverband kombiniert werden. In Fig. 1F ist ein Beispiel gezeigt, in welchem der Verband 100 mit einer Socke S oder Füßling ausgebildet ist. Eine solche Socke ist beispielsweise in der Patentanmeldung DE 10 2016 104 206 A1 beschrieben.

In Fig. 3 sind schematische Schnittmuster zur Herstellung des Verbandes 100 gemäß mehreren Ausführungsbeispielen gezeigt. Auf der linken Seite ist ein Schnittmuster für einen Wadenbereich oder einen Unterschenkel dargestellt. Die jeweiligen resultierenden Schnittkantenabschnitte A1, A2, A3 sind durch Pfeile angedeutet. Das Waden oder Unterschenkelschnittmuster ist jedoch nur beispielhaft. Der Verlauf der Schnittkantenabschnitte A1, A2, A3 kann je nach Schnittmuster variieren. Auf der rechten Seite ist ein Schnittmuster für einen Wadenbereich oder einen Unterschenkel mit Socke S illustriert. Hierbei ist ein weiterer der Socke S zugeordneter Schnittkantenabschnitt A4 gezeigt. Die gestrichelte Linie in den beiden Abbildungen deutet ferner eine resultierende Naht N an, die entsteht, wenn zwei gleichförmige, oder zwei ähnlich geformte, aus dem Schnittmuster erhaltene Zuschnitte Z durch Ultraschallschweißen miteinander verbunden werden.

Der Verbandsteil 10 kann dabei aus zumindest einem Zuschnitt Z eines flachen Werkstoffs hergestellt sein, der einen Vlieswerkstoff umfasst. Hierbei wird vorzugsweise ein Vlieswerkstoff verwendet, der für den Gebrauch in Krankenhäusern und Pflegeheimen vorgesehen ist.

Ein Vlieswerkstoff ist ein Gebilde aus Fasern begrenzter Länge, Endlosfasern (Filamenten) oder geschnittenen Garnen jeglicher Art und jeglichen Ursprungs, die auf irgendeine Weise zu einem Vlies (einer Faserschicht, einem Faserflor) zusammengefügt und miteinander verbunden worden sind.

Als Vlieswerkstoff kann ein Vlieswerkstoff aus synthetischen und/oder natürlichen Fasern bereitgestellt werden. Synthetische Vlieswerkstoffe oder Mischungen von natürlichen und synthetischen Vlieswerkstoffen weisen eine erhöhte Elastizität auf. Aus ökologischen Gründen kann der Vlieswerkstoff nahezu ausschließlich natürliche Fasern umfassen, um ökologisch abbaubar zu sein. Als Fasern können beispielsweise Modalfasern oder Viskosefasern verwendet werden. Ferner weisen natürliche Fasern gegenüber synthetischen Fasern eine bessere Absorptionsfähigkeit auf.

Gemäß der Erfindung kann der Vlieswerkstoff auf einer Rolle und/oder Ballen derart bereitgestellt werden, so dass zumindest zwei Schnittmuster mit entsprechenden Zuschnitten parallel verarbeitet werden können. Ein solcher Zuschnitt Z ist in Fig. 2 und Fig. 4 gezeigt. Entsprechende Schnittmuster sind in Fig. 3 dargestellt. Beispielsweise kann der Vlieswerkstoff vom Vlieshersteller auf einer Rolle, Ballen oder dergleichen geliefert werden. Durch die Parallelverarbeitung mehrerer Schnittmuster zur gleichen Zeit erhöht sich die Anzahl an hergestellten Verbandsteile 10 erheblich ohne dass eine Zunahme an Kosten entsteht.

Die in Fig. 3 dargestellten, schematischen Schnittmuster weisen wie oben beschrieben eine Wadenform oder Unterschenkelform auf. Die illustrierten Schnittmuster sind jedoch nur beispielhaft und es können weitere Formen bereitgestellt werden, insbesondere mit unterschiedlich verlaufenden Schnittkantenabschnitten A1, A1', A2, A2', A3, A3'.

Auch möglich ist, dass der Verbandsteil 10 aus zumindest einem Zuschnitt Z eines Stretch-Materials hergestellt sein kann.

Das Stretch-Material kann hierbei eine Dehnbarkeit von mindestens 50% und ein Rückstellfähigkeit von 30% bis 100% aufweisen.

Die Rückstellung beschreibt, in wie weit sich ein Material wieder in seine ursprüngliche Form folgend einer Dehnung bringt. Der Begriff "Dehnbarkeit" soll hier dem englischen Begriff "Stretch" entsprechen. Hierbei ist ein Material interessant, wenn es dehnbar wie auch teilweise rückstellfähig ist. Für die Verwendung des Verbandes 100 als Einweg-Produkt ist eine volle Rückstellfähigkeit, also ein perfekt elastisches Verhalten nicht notwendig. Das Material muss sich also nach einer Dehnung nicht mehr ganz in seine ursprüngliche Form zurückformen, sondern kann nach einem anfänglichen Kontrahieren in einer gedehnten Form verbleiben. Somit kann die Rückstellfähigkeit kleiner 100% sein, wobei eine Rückstellfähigkeit von 100% einem perfekt elastischen Verhalten entspricht. Erfindungsgemäß bevorzugt ist eine Rückstellfähigkeit von 100% bis 30%, oder von 100% bis 50%, oder von 100% bis 70%. Dehnbarkeit entspricht der Fähigkeit, eine Dehnung ohne Zerstörung wie einem Zerreißen zu überstehen. Je höher die Dehnbarkeit, desto mehr kann das Material prozentual gedehnt werden.

Das Stretch-Material kann einen 2-Wege-Stretch-Stoff aufweisen, wobei sich eine Stretch-Richtung in Quer-Richtung QR des Verbandes 100, d.h., in nur einer Richtung QR erstreckt. Die Quer-Richtung QR liegt hierbei senkrecht auf der Längsrichtung L oder verläuft senkrecht zum Schnittkantenabschnitt A2, und verläuft parallel zu dem Schnittkantenabschnitt des oberen und unteren Bündchenbereichs am Knie bzw. am Knöchel im angelegten Zustand.

Ebenso möglich ist die Verwendung eines 4-Wege-Stretch-Stoffes, wobei sich eine Stretch-Richtung in Querrichtung QR und eine weitere Stretch-Richtung in Längsrichtung LR des Verbandes 100 erstreckt. Je nach Anforderung an den Verband 100 kann der 2-Wege-Stretch-Stoff oder der 4-Wege-Stretch-Stoff verwendet werden. Die Verwendung eines 2-Wege-Stretch- oder Single-Stretch-Materials, dessen Stretch-Richtung sich nur in Querrichtung QR erstreckt, ist jedoch günstiger zu produzieren und weist nur geringe Nachteile gegenüber einem 4-Wege-Stretch-Material auf.

Die in Fig. 2 gezeigten Zuschnitte Z bzw. die daraus hergestellten Verbandsteile 10 sind für einen Beinbereich ausgelegt. Dabei sind Verbandsteile 10 mit unterschiedlicher Bemaßung schematisch illustriert. In der unteren Abbildung ist der aus dem Zuschnitt Z erhaltene Verbandsteil 10 samt Socke S gezeigt. Die jeweiligen Kompressionszonen K1, K2 sind durch die schattierten Flächen hervorgehoben.

Wie oben bereits erwähnt kann das Stretch-Material Elastomer-Fäden oder Elastan-Fäden mit unterschiedlicher Dicke umfassen, die unterschiedliche Kompressionsstärken in den jeweils unterschiedlichen Kompressionszonen K1, K2 erzeugen. Weiterhin kann das Stretch-Material Elastomer-Fäden mit unterschiedlicher Vorspannung umfassen, die unterschiedliche Kompressionsstärken in den jeweils unterschiedlichen Kompressionszonen K1, K2 erzeugen.

Elastanfasern oder Elastanfäden sind synthetische Filamentgarne (Garnfeinheit 11-2600 dtex) und bestehen zu mindestens 85 Gew.-% aus segmentiertem Polyurethan. Sie zeichnen sich besonders durch hohe elastische Dehnung aus. Unter Zugbeanspruchung lassen sie sich bis auf das 6-8-fache ihrer Ausgangslänge dehnen und nehmen nach Aufhebung der Spannung sofort und vollständig wieder ihre ursprüngliche Länge ein. Elastanfasern sind gut färbbar, oxidationsbeständig sowie lichtbeständiger und wesentlich dünner herzustellen als Gummifäden, so dass aus ihnen feinere und leichtere Gewebe und Gewirke als aus Gummifäden erzeugt werden. Als gängiges Herstellungsverfahren dient das Trockenspinnen (80 % der Weltkapazität), Nassspinnen oder Reaktivspinnen. Sie sind widerstandsfähig gegen Schweiß und kosmetische Öle bei sachgemäßer Pflege, gut waschbar, besitzen mäßige Scheuerfestigkeit, eine Dichte von 1,15 g/cm³; eine Feuchtigkeitsaufnahme von 1,0-1,3 %; einen Schmelzpunktbei etwa 250 °C; einen Erweichungspunkt bei 175 °C; bei mehr als 150 °C vergilben sie und werden abgebaut. Die Höchstzugkraft bezogen auf Ausgangsfeinheit beträgt 4,5 cN/tex - 12 cN/tex, die Höchstzugkraftdehnung beträgt 420% -800%, die Restdehnung nach 300 % Dehnung beträgt 10%-30%, die Verklebungstemperatur liegt bei höher 170°C. Markennamen für elastomere Fäden oder Elastanfäden sind Lycra (Invista), Dorlastan (Asahi), creora (Hyosung), oder Linel (Fillatice). Obwohl der Einsatz von Elastanfäden als elastomeren Fäden besonders bevorzugt ist, können selbstverständlich auch andere elastomere Fäden mit vergleichbaren Eigenschaften eingesetzt werden. Das Elastizitätsmodul von Elastanfäden kann hierbei in einem Bereich zwischen 0,01 GPa und 0,1 GPa liegen. Unter Elastizitätsmodul des Verbandmaterials soll dabei ein lokales Elastizitätsmodul bzgl. der Querrichtung QR verstanden werden, das durch in dem Trägermaterial wie beispielweise Vlies eingebrachte elastomere Fäden lokal (also innerhalb eines Bereiches oder einer Zone K1, K2 in Längsrichtung L) in dem Trägermaterial erzeugt wird.

Beispielsweise kann eine Fadenstärke in der ersten Kompressionszone K1 in einem Bereich von 500 dtex bis 1000 dtex, oder in einem Bereich von 500 dtex bis 900 dtex, oder in einem Bereich von 500 dtex bis 800 dtex, oder in einem Bereich von 500 bis 700 dtex, oder in einem Bereich von 500 bis 600 dtex, oder in einem Bereich von 600 dtex bis 800 dtex liegen. In der zweiten Kompressionszone K2 kann die Fadenstärke ferner in einem Bereich von 100 dtex bis 200 dtex, oder in einem Bereich von 100 dtex bis 300 dtex, oder in einem Bereich von 100 dtex bis 400 detx, oder in einem Bereich von 100 dtex bis 500 dtex, oder in einem Bereich von 100 dtex bis 600 dtex, oder in einem Bereich von 300 dtex bis 600 dtex liegen. Ferner kann der Fadenabstand in der ersten Kompressionszone in einem Bereich von 0,5 mm bis 1 mm, oder in einem Bereich von 0,1 mm bis 10 mm, oder in einem Bereich von 0.5 mm bis 8 mm, oder in einem Bereich von 1mm bis 7 mm, oder in einem Bereich von 1 mm bis 6 mm, oder in einem Bereich von 1 mm bis 5 mm, oder in einem Bereich von 1 mm bis 4 mm, oder in einem Bereich von 1 mm bis 3 mm, oder in einem Bereich von 1 mm bis 2 mm liegen. In der zweiten Kompressionszone K2 kann dieser in einem Bereich von 4 mm bis 4,5 mm, oder in einem Bereich von 3 mm bis 5 mm, oder in einem Bereich von 3 mm bis 5,5 mm, oder in einem Bereich von 2 mm bis 6 mm, oder in einem Bereich von 1 mm bis 15 mm, oder in einem Bereich von 2 mm bis 12 mm, oder in einem Bereich von 3 mm bis 10 mm, oder in einem Bereich von 4 mm bis 8 mm, oder in einem Bereich von 5 mm bis 8 mm, oder in einem Bereich von 6 mm bis 8 mm liegen. Dies ist jedoch nur beispielhaft und weitere Fadenstärken und Abstände sind möglich. Weiterhin können die Fäden eine Fadenvorspannung von größer 50 %, oder größer 100 %, oder größer 150 %, oder größer 200 %, oder größer 250 %, oder größer 300 %, oder größer 350%, oder größer 400 %, oder größer 450 %, oder größer 500 % und/oder kleiner als 600 % aufweisen.

Wie aus Fig. 1A ersichtlich kann der Verbandsteil 10 neben dem geschlitzten Bereich B1 den schlauchartig geschlossenen, distalen Bereich B2 umfassen, welcher bei einem Anlegen des Verbandes 100 über einen Fuß oder eine Hand des Lebewesens gezogen wird und im angelegten Zustand des Verbandes 100 zu dem Fuß oder der Hand benachbart ist. Der distale Bereich B2 ist in Fig. 1A ebenfalls vereinfacht durch einen entsprechenden Pfeil angedeutet.

Mittels des geschlossenen Bereichs B2 kann der Verband 100 also einfach und bequem angelegt und durch die entsprechenden Verschlusselemente 22, 22' geschlossen werden. Der geschlossene Bereich wird erst an einer entsprechende Stelle (keine Wundstelle) positioniert, beispielsweise an einem Knöchel oder an einem Handgelenk, dann kann der lose geschlitzte Teil oder der lose Lappen des geschlitzten Bereichs B1 des Verbandsteils 10 mittels der Verschlusselemente 22, 22' einfach lösbar geschlossen werden. Hierdurch wird das Anlegen des Verbandes 100 weiter vereinfacht.

Der Verbandsteil 10 kann dabei in einem angelegten Zustand in seinem schlauchartig geschlossenen, distalen Bereich B2 einen Knöchelbereich eines Fußes und in seinem geschlitzten Bereich B1 einen Wadenbereich eines Unterschenkels komprimierend umschließen. Beispielsweise kann der Verband 100 dabei als biokompatible physikalische Verbandschicht fungieren, der die Haut eines Lebewesens schützt und unterstützt.

Wie bereits oben beschrieben kann der Verschlussteil 20 als Verschlusselemente 22, 22' einzelne, aus einem Klettverschlussband ausgestanzte Klettverschlussteile umfassen, die einzeln mit dem Verbandsteil 10 verbunden sind.

In einer weiteren Ausführungsform kann der Verschlussteil 20 auch als einstückiges Multi-Klettverschlussteil ausgebildet sein, das auf seiner einen Seite in seinem Nicht-Klettbereich mit dem Verbandsteil 10 verbunden ist und auf seiner gegenüberliegenden Seite in seinem Klettbereich vorspringende Verschlusselemente 22, 22' aufweist, die durch Stanzen des Klettverschlussbandes ausgebildet sind.

Die lösbaren Verschlusselemente 22, 22' ermöglichen es den Verband 100 beliebig zu öffnen, beispielsweise um einen Heilungsprozess einer Wunde zu kontrollieren oder eine entsprechende Wundauflage, wie ein Wundpflaster oder ein Pad einfach auszuwechseln. Hierbei ist kein unnötiges Wickeln wie bei herkömmlichen Verbänden erforderlich.

Die oben beschriebenen Verschlusselemente 22, 22' können statt oder neben den oben beschriebenen Klettverschlusselementen Hakenverschlusselemente, oder Klebebandverschlusselemente (zum Beispiel mit Abziehfolie) aufweisen.

Ein solcher Multi-Klettverschlussteil ist beispielsweise in Fig. 4 dargestellt, wobei das Ausstanzen der Verschlusselemente 22 vereinfacht dargestellt ist. In Fig. 4 sind zwei übereinander angeordnete Zuschnitte Z gezeigt, die aus einem Vlieswerkstoff oder Stretch-Material entnommen und anschließend schrittweise weiter verarbeitet werden.

Gemäß einem Verfahren zum Herstellen des Verbandes 100 kann der Vlieswerkstoff oder das Stretch-Material wie oben bereits beschrieben auf einer Rolle und/oder Ballen bereitgestellt werden, wobei zwei Teilstücke aus dem Vlieswerkstoff oder dem Stretch-Material gemäß einem vorgegebenen Schnittmuster, wie es mit Bezug auf die Fig. 3 beschrieben wurde, entnommen werden können. Die Entnahme der Teilstücke aus dem Vlieswerkstoff oder dem Stretch-Material kann durch Schneiden oder Stanzen erfolgen. Die dabei erhaltenen gleichförmigen Zuschnitte Z können zeitgleich an entsprechenden Endbereichen durch Ultraschallschweißen miteinander verbunden werden. Beispielsweise können die Zuschnitte Z mittels einer in Nahtrichtung N elastischen Naht miteinander verbunden sein. Die Nahtrichtung N ist in Fig. 1A, 1B, 1F und Fig. 3 als gestrichelte Linie illustriert.

Anschließend können die Zuschnitte Z weiter verarbeitet werden, wobei in einem folgenden Bearbeitungsschritt an den Endbereichen Vlieswerkstoff oder Stretch-Material ausgespart wird. Zum Beispiel kann an einer in Fig. 4 dargestellten oberen Seite des Zuschnitts Z anstelle des ausgesparten Materials ein dem Verschlussteil 20 zugeordnetes Material mit dem Zuschnitt Z verbunden werden. Aus diesem können dann die Verschlusselemente 22 ausgestanzt werden, um den Verbandsteil 10 zu bilden.

In einer weiteren Ausführungsform kann ein Zuschnitt Z auch einstückig entnommen und verarbeitet werden. Hierbei kann beispielsweise an einem seitlichen Ende der Verschlussteil 20 angebracht werden. Der Verbandsteil 100 kann dann durch Falten des Zuschnitts Z gebildet werden. Hierbei erstreckt sich der geschlitzte Bereich B1 entlang der gesamten Längsrichtung LR. Eine resultierende Faltlinie erstreckt sich in Längsrichtung LR auf der dem Verschlußteil 20 gegenüberliegenden Seite. In diesem Fall ist jedoch diese Seite nicht gekrümmt und an eine Wadenform angepasst, sondern ist idealerweise gerade, um ein einfaches Falten zu gewährleisten.

Wie mit Bezug auf Fig. 3 beschrieben wurde, kann der Verbandsteil 10 eine dem Wadenbereich eines Unterschenkels angepasste Form aufweisen. Hierdurch kann der Verband 100 eine optimale Passform von Anfang an bereitstellen. Weitere Formen sind möglich. Ebenso kann auch der Füßling oder die Socke S der weiteren Ausführungsform eine entsprechend an einem Fuß angepasste Form bereitstellen.

Wie bereits beschrieben kann der Verband 100 als hygienisches Einweg-Produkt ausgebildet sein.

Der erfindungsmäßige Verband 100 stellt einen anpassungsfähigen, gebrauchsfertigen Unterschenkelverband (zum Einmalgebrauch) bereit, der den Unterschenkel vom Knöchel bis unter das Knie bedeckt. Ein Zweck des erfindungsmäßigen Verbandes 100 ist es, als biokompatible physikalische Schicht zu wirken, die Haut zu schützen und zu unterstützen. Der Verband 100 ist dazu bestimmt, allein oder in Verbindung mit anderen Produkten zur medizinischen Behandlung verwendet zu werden. Bei der Anwendung auf verletzter Haut dient es als sekundäre oder tertiäre Verbandschicht um den Unterschenkel. Der Verband 100 hat polsternde Eigenschaften, kann zum Schutz darunterliegender Schichten (z.B. Wundauflagen) beitragen und überschüssige Flüssigkeit begrenzt absorbieren. Der Verband 100 ist ein unsteriles, nicht-aktives, nicht-invasives Produkt.

Der erfindungsgemäße Verband 100 ist ferner für die Anwendung unter Kompression geeignet und ist mit einem gebrauchsfertigen Fußverband gut kombinierbar.

Beispielsweise kann der Verband 100 bei Patienten angewendet werden, welche eine kurz- oder langfristige medizinische Behandlung des Unterschenkels erhalten oder bettlägerig oder eingeschränkt mobil sind.

Im Folgenden wird ein Anwendungsbeispiel des erfindungsgemäßen Verbands 100 für den rechten oder linken Unterschenkel erläutert.

Zuerst erfolgt eine Auswahl des Verbandes 100 anhand einer Größentabelle. Bei der Auswahl gilt es die folgenden Punkte zu beachten. Die Dicke einer Wundauflage muss berücksichtigt werden. Ebenso sind Änderungen des Unterschenkelumfangs im Tagesverlauf durch Ödembildung und -rückbildung möglich. Im Heilungsverlauf oder durch Veränderung der Versorgungsart kann die Verwendung einer anderen Produktgröße notwendig werden. Vor dem Anlegen muss eine fachgerechte Versorgung vorhandener Wunden erfolgen. Der Verband 100 sollte am unteren Bund vorsichtig vorgedehnt werden (dabei an den Nahtstellen halten, um ein Aufreißen zu vermeiden). Der Verband 100 muss so angelegt werden, dass die Öffnung mit Klettverschlüssen nach vorne zeigt. Den unteren Bund dann vorsichtig über den Fuß stülpen und bis zum Knöchel ziehen. Dabei sind die Laschen der Öffnung nahe am Nahtende zu greifen. Gegebenenfalls kann der Bund mit der Hand von innen aufgespreizt werden, um Reibung am Fuß zu reduzieren. Die Verschlüsse sind der Reihe nach vorzufixieren, dabei von unten nach oben arbeiten. Darauf achten, dass die beiden Seiten der Öffnung ausreichend überlappen. Anschließend die Verschlüsse nachjustieren. Der Verband 100 sollte glatt anliegen und keine Falten aufweisen. Nach Gebrauch Abziehen und Entsorgen. Weiterhin sollten die Klettverschlüsse nachjustiert werden (lockern/weiten), wenn (a) das Gefühl von Einschnürung entsteht, (b) der Verband 100 an einer Stelle keine Elastizität mehr aufweist, bzw. stark gespannt ist, (c) der Verband 100 rutscht. Anwendung des Verbandes 100 als Primärlage: Auf intakter, unverletzter Haut. Anwendung des Verbandes 100 als Sekundärlage: Auf verletzter Haut oder offenen Wunden mit darunterliegender Primärlage (Wundauflage).

## Patentansprüche

1. Verband (100) zum Umschließen eines Bein- oder Armbereiches eines Lebewesens, mit:
einem länglichen, schlauchförmigen Verbandsteil (10), der zumindest teilweise in Längsrichtung (LR) einfach geschlitzt ist, und der dazu angepasst ist, in einem angelegten Zustand den Bein- oder Armbereich elastisch zu umschließen; und
einem Verschlussteil (20) mit Verschlusselementen (22), der mit einem Ende des geschlitzten Bereiches (B) des Verbandsteils (10) fest verbunden ist, um den geschlitzten Bereich (B) durch überlappende Verbindung der benachbarten Enden des geschlitzten Bereiches (B) mittels der Verschlusselemente (22, 22') lösbar zu verschließen, **dadurch gekennzeichnet, dass**
der Verbandsteil (10) in Längsrichtung (LR) unterschiedliche Kompressionszonen (K1, K2) mit jeweils unterschiedlichen radialen Kompressionsstärken quer zur Längsrichtung (L) aufweist,
wobei der Verbandsteil (10) aus zumindest einem Zuschnitt (Z) eines Stretch-Materials hergestellt ist,
und wobei das Stretch-Material
- Elastomer-Fäden mit unterschiedlicher Dicke umfasst, die unterschiedliche Kompressionsstärken in den jeweils unterschiedlichen Kompressionszonen (K1, K2) erzeugen,
- und/oder wobei das Stretch-Material Elastomer-Fäden mit unterschiedlicher Vorspannung umfasst, die unterschiedliche Kompressionsstärken in den jeweils unterschiedlichen Kompressionszonen (K1, K2) erzeugen.

2. Verband (100) nach Anspruch 1, **dadurch gekennzeichnet, dass** die unterschiedlichen Kompressionszonen (K1, K2) vorbestimmten Bereichen eines Schenkels, insbesondere eines Unterschenkels, oder eines Arms, insbesondere eines Unterarms, zugeordnet sind.

3. Verband (100) nach Anspruch 2, **dadurch gekennzeichnet, dass** eine erste Kompressionszone (K1) in dem Verbandsteil (10) einem Bundbereich am Knie und/oder am Knöchel des Unterschenkels zugeordnet ist, und dass eine zweite Kompressionszone (K2) in dem Verbandsteil (10) einem Wadenbereich des Unterschenkels zugeordnet ist, wobei die erste Kompressionszone (K1) eine höhere Kompressionsstärke aufweist als die zweite Kompressionszone (K2).

4. Verband (100) nach einem der vorhergehenden Ansprüche, **dadurch gekennzeichnet, dass** der Verbandsteil (10) aus zumindest einem Zuschnitt (Z) eines flachen Werkstoffs hergestellt ist, der einen Vlieswerkstoff umfasst, und/oder als hygienisches Einweg-Produkt ausgebildet ist.

5. Verband (100) nach einem der vorhergehenden Ansprüche, **dadurch gekennzeichnet, dass** das Stretch-Material eine Dehnbarkeit von mindestens 50% und ein Rückstellfähigkeit von 30% bis 100% aufweist.

6. Verband (100) nach einem der vorhergehenden Ansprüche, **dadurch gekennzeichnet, dass** das Stretch Material einen 2-Wege-Stretch-Stoff aufweist, wobei sich eine Stretch-Richtung in Quer-Richtung (QR) des Verbandes (100) erstreckt.

7. Verband (100) nach einem der vorhergehenden Ansprüche, **dadurch gekennzeichnet, dass** das Stretch- Material einen 4-Wege-Stretch-Stoff aufweist, wobei sich eine Stretch-Richtung in Querrichtung (QR) und eine weitere Stretch-Richtung in Längsrichtung (LR) des Verbandes (100) erstreckt.

8. Verband (100) nach einem der vorhergehenden Ansprüche, **dadurch gekennzeichnet, dass** der Verbandsteil (10) neben dem geschlitzten Bereich (B1) einen schlauchartig geschlossenen, distalen Bereich (B2) umfasst, welcher bei einem Anlegen des Verbandes.(100) über einen Fuß oder eine Hand des Lebewesens gezogen wird und im angelegten Zustand des Verbandes (100) zu dem Fuß oder der Hand benachbart ist.

9. Verband (100) nach Anspruch 8, **dadurch gekennzeichnet, dass** der Verbandsteil (10) in einem angelegten Zustand in seinem schlauchartig geschlossenen, distalen Bereich (B2) einen Knöchelbereich eines Fußes und in seinem geschlitzten Bereich (B1) einen Wadenbereich eines Unterschenkels komprimierend umschließt und/oder eine dem Wadenbereich eines Unterschenkels angepasste Form aufweist.

10. Verband (100) nach einem der vorstehenden Ansprüche, **dadurch gekennzeichnet, dass** der Verschlussteil (20) als Verschlusselemente (22, 22') einzelne, aus einem Klettverschlussband ausgestanzte Klettverschlussteile umfasst, die einzeln mit dem Verbandsteil (10) verbunden sind.

11. Verband (100) nach einem der Ansprüche 1 bis 9, **dadurch gekennzeichnet, dass** der Verschlussteil (20) als einstückiges Multi-Klettverschlussteil ausgebildet ist, das auf seiner einen Seite in seinem Nicht-Klettbereich mit dem Verbandsteil (10) verbunden ist und auf seiner gegenüberliegenden Seite in seinem Klettbereich vorspringende Verschlusselemente (22, 22') aufweist, die durch Stanzen des Klettverschlussbandes ausgebildet sind.

12. Verband (100) nach einem der vorhergehenden Ansprüche, **dadurch gekennzeichnet, dass** der Verbandsteil (10) aus zwei gleichförmigen Zuschnitten (Z) hergestellt ist, die mittels einer in Nahtrichtung (N) elastischen Naht miteinander verbunden sind.

## Claims

1. Bandage (100) for enclosing a leg region or arm region of a living being, having:
an elongate, tubular bandage part (10) which is at least partially slit once in the longitudinal direction (LR) and which in an applied state is adapted to elastically enclose the leg region or arm region; and
a fastener part (20) which has fastener elements (22) and is fixedly connected to one end of the slit region (B) of the bandage part (10) so as to releasably close the slit region (B) by way of an overlapping connection of the adjacent ends of the slit region (B) by means of the fastener elements (22, 22'), **characterized in that**
the bandage part (10) in the longitudinal direction (LR) has dissimilar compression zones (K1, K2) having in each case dissimilar radial compression strengths transverse to the longitudinal direction (L),
wherein the bandage part (10) is produced from at least one blank (Z) of a stretch material,
and wherein the stretch material
- comprises elastomer threads of dissimilar thicknesses which generate dissimilar compression strengths in the respective dissimilar compression zones (K1, K2),
- and/or wherein the stretch material comprises elastomer threads with dissimilar preloads which generate dissimilar compression strengths in the respective dissimilar compression zones (K1, K2).

2. Bandage (100) according to Claim 1, **characterized in that** the dissimilar compression zones (K1, K2) are assigned to predetermined regions of a leg, in particular of a lower leg, or of an arm, in particular of a lower arm.

3. Bandage (100) according to Claim 2, **characterized in that** a first compression zone (K1) in the bandage part (10) is assigned to a collar region on the knee and/or on the ankle of the lower leg, and **in that** a second compression zone (K2) in the bandage part (10) is assigned to a calf region of the lower leg, wherein the first compression zone (K1) has a higher compression strength than the second compression zone (K2).

4. Bandage (100) according to one of the preceding claims, **characterized in that** the bandage part (10) is produced from at least one blank (Z) of a flat material which comprises a non-woven material and/or is configured as a hygienic single-use product.

5. Bandage (100) according to one of the preceding claims, **characterized in that** the stretch material has an elasticity of at least 50% and a resilience of 30% to 100%.

6. Bandage (100) according to one of the preceding claims, **characterized in that** the stretch material has a 2-way stretch fabric, wherein one stretch direction extends in the transverse direction (QR) of the bandage (100).

7. Bandage (100) according to one of the preceding claims, **characterized in that** the stretch material has a 4-way stretch fabric, wherein one stretch direction extends in the transverse direction (QR) and a further stretch direction extends in the longitudinal direction (LR) of the bandage (100).

8. Bandage (100) according to one of the preceding claims, **characterized in that** the bandage part (10) next to the slit region (B1) comprises a distal region (B2) which is closed in a tubular manner and when applying the bandage (100) is pulled over a foot or a hand of the living being, and in the applied state of the bandage (100) is adjacent to the foot or the hand.

9. Bandage (100) according to Claim 8, **characterized in that** the bandage part (10) in an applied state, in its distal region (B2) which is closed in a tubular manner, encloses in a compressing manner an ankle region of a foot, and in its slit region (B1) encloses in a compressing manner a calf region of a lower leg and/or has a shape adapted to the calf region of a lower leg.

10. Bandage (100) according to one of the preceding claims, **characterized in that** the fastener part (20) as fastener elements (22, 22') comprises individual hook and loop fastener parts which are punched out of a hook and loop fastener tape and are individually connected to the bandage part (10).

11. Bandage (100) according to one of Claims 1 to 9, **characterized in that** the fastener part (20) is configured as an integral multi-hook and loop fastener part which on the one side thereof, in its non-hook region, is connected to the bandage part (10), and on the opposite side thereof, in its hook region, has projecting fastener elements (22, 22') which are configured by punching the hook and loop fastener tape.

12. Bandage (100) according to one of the preceding claims, **characterized in that** the bandage part (10) is produced from two blanks (Z) of identical shape, which are connected to one another by a seam which is elastic in a seam direction (N).

## Revendications

1. Bandage (100) pour entourer une zone de la jambe ou du bras d'un être vivant, avec :
une partie de bandage tubulaire allongée (10) qui est au moins partiellement fendue une fois dans la direction longitudinale (LR) et qui est adaptée pour entourer de manière élastique la zone de la jambe ou du bras dans un état mis en place ; et
une partie de fermeture (20) avec des éléments de fermeture (22), qui est reliée de manière fixe à une extrémité de la zone fendue (B) de la partie de bandage (10), afin de fermer de manière amovible la zone fendue (B) en reliant par chevauchement les extrémités voisines de la zone fendue (B) au moyen des éléments de fermeture (22, 22'), **caractérisé en ce que**
la partie de bandage (10) présente, dans la direction longitudinale (LR), différentes zones de compression (K1, K2) avec respectivement différentes intensités de compression radiale transversalement à la direction longitudinale (L),
la partie de bandage (10) étant fabriquée à partir d'au moins une découpe (Z) d'un matériau extensible,
et le matériau extensible
- comprenant des fils élastomères de différentes épaisseurs qui produisent différentes intensités de compression dans les différentes zones de compression respectives (K1, K2),
- et/ou le matériau extensible comprenant des fils élastomères avec différentes précontraintes qui produisent différentes intensités de compression dans les différentes zones de compression respectives (K1, K2).

2. Bandage (100) selon la revendication 1, **caractérisé en ce que** les différentes zones de compression (K1, K2) sont associées à des zones prédéterminées d'une cuisse, notamment d'une jambe, ou d'un bras, notamment d'un avant-bras.

3. Bandage (100) selon la revendication 2, **caractérisé en ce qu'**une première zone de compression (K1) dans la partie de bandage (10) est associée à une zone de ceinture au niveau du genou et/ou de la cheville de la jambe, et **en ce qu'**une deuxième zone de compression (K2) dans la partie de bandage (10) est associée à une zone de mollet de la jambe, la première zone de compression (K1) présentant une intensité de compression plus élevée que la deuxième zone de compression (K2).

4. Bandage (100) selon l'une quelconque des revendications précédentes, **caractérisé en ce que** la partie de bandage (10) est fabriquée à partir d'au moins une découpe (Z) d'un matériau plat comprenant un matériau non tissé et/ou est réalisée sous forme de produit hygiénique à usage unique.

5. Bandage (100) selon l'une quelconque des revendications précédentes, **caractérisé en ce que** le matériau extensible présente une extensibilité d'au moins 50 % et une reprise élastique de 30 % à 100 %.

6. Bandage (100) selon l'une quelconque des revendications précédentes, **caractérisé en ce que** le matériau extensible présente un tissu extensible à 2 voies, une direction d'extension s'étendant dans la direction transversale (QR) du bandage (100).

7. Bandage (100) selon l'une quelconque des revendications précédentes, **caractérisé en ce que** le matériau extensible présente un tissu extensible à 4 voies, une direction d'extension s'étendant dans la direction transversale (QR) et une autre direction d'extension s'étendant dans la direction longitudinale (LR) du bandage (100).

8. Bandage (100) selon l'une quelconque des revendications précédentes, **caractérisé en ce que** la partie de bandage (10) comprend, outre la zone fendue (B1), une zone distale (B2) fermée sous forme tubulaire, qui est tirée sur un pied ou une main de l'être vivant lors d'une mise en place du bandage (100) et qui est voisine du pied ou de la main à l'état mis en place du bandage (100).

9. Bandage (100) selon la revendication 8, **caractérisé en ce que** la partie de bandage (10), dans un état mis en place, entoure de manière compressive, dans sa zone distale (B2) fermée sous forme tubulaire, une zone de cheville d'un pied et, dans sa zone fendue (B1), une zone de mollet d'une jambe et/ou présente une forme adaptée à la zone de mollet d'une jambe.

10. Bandage (100) selon l'une quelconque des revendications précédentes, **caractérisé en ce que** la partie de fermeture (20) comprend, en tant qu'éléments de fermeture (22, 22'), des parties de fermeture auto-agrippante individuelles découpées dans une bande de fermeture auto-agrippante, qui sont reliées individuellement à la partie de bandage (10).

11. Bandage (100) selon l'une quelconque des revendications 1 à 9, **caractérisé en ce que** la partie de fermeture (20) est réalisée sous forme de partie de fermeture auto-agrippante multiple monobloc, reliée d'un côté à la partie de bandage (10) dans sa zone non auto-agrippante, et présentant sur son côté opposé, dans sa zone auto-agrippante, des éléments de fermeture (22, 22') en saillie, réalisés par découpage de la bande de fermeture auto-agrippante.

12. Bandage (100) selon l'une quelconque des revendications précédentes, **caractérisé en ce que** la partie de bandage (10) est fabriquée à partir de deux découpes (Z) de même forme, qui sont reliées entre elles au moyen d'une couture élastique dans la direction de la couture (N).
